# NEW EUROPEAN PATENT SPECIFICATION

(11) **EP 1 758 469 B2**
(45) Date of publication and mention of the opposition decision: **25.09.2013**
(45) Mention of the grant of the patent: 28.10.2009
(21) Application number: 05755280.4
(22) Date of filing: 22.06.2005
(51) Int. Cl.: A23L 1/30, A23L 1/308, A61P 31/18, A61K 31/201, A61K 31/202, A61K 45/06, A61K 31/702

(54) **IMPROVEMENT OF BARRIER INTEGRITY IN HIV PATIENTS BY USE OF FATTY ACIDS**
VERBESSERUNG DER BARRIEREINTEGRITÄT BEI HIV-PATIENTEN MIT HILFE VON FETTSÄUREN
AMELIORATION DE L'INTEGRITE DE LA BARRIERE INTESTINALE CHEZ LES PATIENTS ATTEINTS DE VIH

(30) Priority: 22.06.2004 WO PCT/NL2004/000444; 21.04.2005 EP 05103257; 21.04.2005 EP 05103260
(43) Date of publication of application: 07.03.2007
(73) Proprietor: N.V. Nutricia, 2712 HM Zoetermeer (NL)
(72) Inventor: VAN TOL, Eric, Alexander, Franciscus, NL-6824 MZ Arnhem (NL); WILLEMSEN, Linette, Eustachia, Maria, NL-3522 AJ Utrecht (NL); KOETSIER, Marleen, Antoinette, NL-8161 DW EPE (NL); BEERMANN, Christopher, 61267 Neu-Anspach (DE); STAHL, Bernd, 61191 Rosbach (DE)
(74) Representative: Nederlandsch Octrooibureau
(86) International application number: PCT/NL2005/000451
(87) International publication number: WO 2005/122791

(56) References cited:
- EP-A- 0 378 824
- EP-A- 0 615 752
- EP-A- 0 641 562
- EP-A- 1 454 990
- EP-A2- 0 626 177
- WO-A-99/53777
- WO-A-2004/026294
- WO-A-2004/112509
- WO-A-2005/122790
- DE-A1- 10 136 260
- US-A- 5 792 754
- DATABASE WPI Section Ch, Week 199615 Derwent Publications Ltd., London, GB; Class B04, AN 1996-145913 XP002364978 & JP 08 033448 A (MEIJI MILK PROD CO LTD) 6 February 1996 (1996-02-06)
- DATABASE WPI Section Ch, Week 198929 Derwent Publications Ltd., London, GB; Class B03, AN 1989-211474 XP002365136 & JP 01 149730 A (OSAKA PREFECTURE) 12 June 1989 (1989-06-12)

## Description

### FIELD OF THE INVENTION

The present invention relates to the use of a composition for the manufacture of a medicament for improving intestinal barrier integrity of HIV patients and a composition suitable for use in such method.

### BACKGROUND OF THE INVENTION

The gastrointestinal epithelium normally functions as a selective barrier permitting the absorption of nutrients, electrolytes and water and preventing the exposure to dietary and microbial antigens, including food allergens. The gastrointestinal epithelium limits the passage of antigens to the systemic circulation that may be causing inflammatory reactions, e.g. allergic reactions. As the incidence of allergy, particularly food allergy, is increasing, many research groups search for (preventive) cures for these ailments.

EP1272058 describes a composition containing indigestible oligosaccharides for improving tight junction to reduce intestinal permeability and reducing allergic reaction. The composition may comprise LC-PUFA's (long chain-polyunsaturated faty acids).

EP 745001 describes a combination of indigestible oligosaccharides and n-3 and n-6 fatty acids for treatment ulcerative colitis.

Usami et al (Clinical Nutrition 2001, 20(4): 351-359) describe the effect of eicosapentaenoic acid (EPA) on tight junction permeability in intestinal monolayer cells. In their hands, EPA was found to increase permeability, indicating that EPA is unsuitable to improve intestinal barrier integrity.

WO 2005/039597 describes a composition comprising acid oligosaccharides and neutral oligosaccharides for enhancing the immune system in mammals.

The prior art formulations are not optimally suited for improving barrier integrity.

### SUMMARY OF THE INVENTION

The present invention provides a combination of selected long chain polyunsaturated fatty acids (LC-PUFA's) and selected oligosaccharides. The present combination of LC-PUFA's and oligosaccharides effectively improves barrier integrity, by synergistically improving intestinal permeability, mucus production, and reducing the mucosal production of inflammatory mediators that could compromise intestinal barrier integrity. A combination of these compounds in a nutritional or pharmaceutical formulation is particularly suitable for improving intestinal integrity in HIV and AIDS patients.

It was surprisingly found that selected LC-PUFA's effectively reduce epithelial paracellular permeability. In contrast to what Usami et al (Clinical Nutrition 2001, 20(4): 351-359) have reported, the present inventors found that C18 and C20 polyunsaturated fatty acids, particularly eicosapentaenoic acid (EPA), docosahexaenoic acid (DHA) and arachidonic acid (ARA), are capable of effectively reducing intestinal tight junction permeability.

In addition to the LC-PUFAs, the present composition contains oligosaccharides. The selected oligosaccharides improve the barrier integrity by stimulating the production of the mucus, which results in an increased mucus layer thickness. It is believed this effect is caused by the effects of the distinct oligosaccharides on the short chain fatty acid (SCFA) production. Hence, when enterally administered to a mammal, the present combination of LC-PUFA and indigestible oligosaccharides synergistically improve barrier integrity and/or synergistically reduce intestinal permeability by simultaneous reduction of tight junction permeability and stimulation of mucus production.

In a further aspect, the present composition improves the quality of the intestinal mucus layer. The mucus layer comprises mucins. Mucins are high molecular mass glycoproteins that are synthesized and secreted by goblet cells. They form a gel-like layer on the mucosal surface, thereby improving barrier integrity. The mucus layer comprises different types of mucins, e.g. acid, neutral and sulphonated mucins. An increased heterogeneity of the mucus layer is believed to improve barrier functionality.

The present composition preferably comprises at least two different oligosaccharides, which influence the mucosal architecture and advantageously influence mucin heterogeneity in the mucus layer, either directly or by changing the intestinal flora. Each different selected oligosaccharide is believed to have a different effect on mucus quantity and quality. Moreover, the two distinct oligosaccharides are also able to stimulate quality of mucus as reflected by the degree of sulphation through their synergistic stimulation of SCFA production. It was surprisingly found by the present inventors that a mixture of two different oligosaccharides according to the present invention synergistically stimulates acetate production. It was also found by the present inventors mucus production is dependent on acetate production.

It was further found that the precursor of ARA, gamma linolenic acid (GLA), can be advantageously combined in the present composition for the treatment of HIV patients. HIV patients often suffer from intestinal inflammatory disorders, which makes a high intake of ARA undesirable, because ARA increases inflammatory response. It was found that part of the ARA can be replaced by GLA without having a negative effect on the effectiveness of the fat blend. Hence, in a further aspect, the present invention provides a composition comprising the oligosaccharides, EPA, DHA, ARA and GLA and the use thereof in the treatment and/or prevention of HIV or AIDS.

The present composition is preferably further improved by providing both long- and short-chain oligosaccharides. The supply of different chain lengths results in stimulation of mucus production in different parts of the ileum and colon. The short chain oligosaccharides (typically with a degree of polymerisation (DP) of 2,3,4 or 5) stimulate mucin production in the proximal colon and/or distal ileum, while the oligosaccharides with longer chain lengths (preferably with a degree of polymerisation (DP) of more than 5 up to 60) are believed to stimulate mucin production in the more distal parts of the colon.

Even further improvements can be achieved by providing the at least two different oligosaccharides both as short-chain and long-chain oligosaccharides. These preferred embodiments all contribute to further improved barrier integrity throughout the ileum and/or colon.

Furthermore, it was surprisingly found that EPA, DHA, and ARA were able to reduce the harmful effects of interleukin 4 (IL-4) on intestinal permeability. IL-4 is a cytokine which is secreted in increased amounts by mucosal T-cells in certain patients and induces intestinal permeability. Hence the present invention also provides for a method for the treatment and/or prevention of diseases wherein intestinal IL-4 concentration is increased, such as allergy, particularly atopic dermatitis.

### DETAILED DESCRIPTION OF THE INVENTION

The present invention relates to a nutritional composition comprising:
a) EPA, DHA and ARA, wherein the content of long chain polyunsaturated fatty acid with 20 and 22 carbon atoms is at least 15% and does not exceed 85 wt.% of the total fat content and wherein the ARA content is at least 0.1 wt% of the total fat; and
b) at least two distinct oligosaccharides, wherein the two distinct oligosaccharides have a homology in monose units below 90 %.
This composition can be advantageously used in a method for stimulating intestinal barrier integrity, said method comprising administering to a mammal said composition.

In a further aspect the present invention provides the use of polyunsaturated fatty acids for the manufacture of a composition for use in a method for the treatment of a patient infected with human immunodeficiency virus (HIV), said method comprising administering to said patient infected with human immunodeficiency virus (HIV) a composition comprising:
a. eicosapentaenoic acid (EPA), docosahexaenoic acid (DHA) and arachidonic acid (ARA), wherein the content of long chain polyunsaturated fatty acid with 20 and 22 carbon atoms is at least 15% and does not exceed 85 wt.% of the total fat content and wherein the ARA content is at least 0.1 wt% of the total fat; and
b. at least two distinct oligosaccharides (OL1 and OL2), wherein the two distinct oligosaccharides have a homology in monose units below 90 %.

A particular embodiment of the treatment of a patient infected with HIV is the nutritional treatment. Other embodiments of the present invention are the use of the composition defined above for providing nutrition to patient infected with HIV, by administering to said patient infected with HIV said composition and also the use of the composition defined above for stimulating intestinal barrier integrity in a patient infected with HIV, by comprising administering to said patient infected with HIV said composition.

### Polyunsaturated fatty acids

The present inventors surprisingly found that eicosapentaenoic acid (EPA, n-3), docosahexaenoic acid (DHA, n-3) and arachidonic acid (ARA, n-6) effectively reduce intestinal tight junction permeability. GLA (n-6) also effectively reduces barrier permeability. Hence the present composition, which is particularly suitable for improving intestinal barrier integrity, comprises EPA, DHA and ARA optionally combined with GLA.

The present inventors found that selected LC-PUFA's, were effective in reducing tight junction permeability (see Examples vs. Usami et al). The content of LC-PUFA with 20 and 22 carbon atoms in the present composition, preferably does not exceed 85 wt.% of the total fat content, preferably does not exceed 35 wt.% of the total fat content. The present composition comprises at least 15 wt% and most preferably at least 25 wt.% LC-PUFA with 20 and 22 carbon atoms of the total fat content. For the same reason, the EPA content preferably does not exceed 55 wt.% of the total fat, preferably does not exceed 35 wt.%, more preferably does not exceed 25 wt.%, but is preferably at least 0.05 wt%, more preferably at least 0.1 wt.% and most preferably at least 1% of the total fat. The DHA content preferably does not exceed 15 wt.%, more preferably does not exceed 10 wt.%, but is at least 0.1 wt% of the total fat. As ARA was found to be particularly effective in reducing tight junction permeability, the present composition comprises relatively high amounts, at least 0.1 wt.%, preferably at least 0.25 wt.%, most preferably at least 0.5 wt.% of the total fat. The ARA content preferably does not exceed 15 wt.%, preferably does not exceed 5 wt%, more preferably does not exceed 1 wt.% of the total fat. In the present ARA containing enteral composition, EPA and DHA are advantageously added to balance the action of ARA, e.g. reduce the potential proinflammatory action of ARA metabolites. Excess metabolites from ARA may cause inflammation. The present nutritional composition preferably also contains gamma-linolenic acid (GLA, C18). The GLA acts as a precursor of ARA, to replace at least in part the ARA content of the composition in order to further decrease the pro-inflammatory effect of ARA.

Hence, the present composition preferably comprises ARA, GLA, EPA and DHA, wherein the weight ratio (ARA+GLA)/DHA preferably is above 0.10, preferably above 0.25 and more preferably above 0.5. The ratio is preferably below 25, and most preferably below 3.
The present composition preferably comprises between 15 and 75 wt.% polyunsaturated fatty acids based on total fat, preferably between 15 and 50 wt.%.

The LC-PUFA with 20 and 22 carbon atoms may be provided as free fatty acids, in triglyceride form, in phospholipid form, or as a mixture of one of more of the above. The present composition preferably comprises at least one of ARA and DHA in phospholipid form.

The present nutritional composition preferably also provides omega-9 (n-9) fatty acid (preferably oleic acid, 18:1), to provide sufficient nutrition. Preferably the present composition provides at least 1 wt.% n-9 fatty acid based on the weight of the total fatty acids, more preferably at least 5 wt.%. The content of n-9 fatty acids is preferably below 80 wt.%.

Suitable daily amount may be at least 0.1 gram EPA and 0.05 gram ARA or ARA + GLA, or between 0.1 and 5 gram EPA and between 0.05 and 2.5 gram ARA or ARA + GLA, or between 0.5 and 2.5 gram EPA and between 0.25 and 1.25 gram ARA or ARA + GLA or an amount between 0.75 and 1.5 gram EPA and between 0.37 and 0.75 gram ARA or ARA + GLA. Suitable daily amounts for DHA follow from the ratio (ARA + GLA)/DHA given above.

### Oligosaccharides

Suitable oligosaccharides according to the invention are saccharides which have a degree of polymerisation (DP) of at least 2 monose units, which are not or only partially digested in the intestine by the action of acids or digestive enzymes present in the human upper digestive tract (small intestine and stomach), but which are fermentable by the human intestinal flora. The term monose units refers to units having a closed ring structure, preferably hexose, e.g. the pyranose or furanose forms. The degree of polymerisation of the oligosaccharide is typically below 60 monose units, preferably below 40, even more preferably below 20.

The present composition comprises at least two different oligosaccharides, wherein the oligosaccharides have a homology in monose units below 90%, preferably below 50%, even more preferably below 25%, even more preferably below 5%. The term "homology" as used in the present invention is the cumulative of the percentage of same monose units in the different oligosaccharides. For example, oligosaccharide 1 (OL1) has the structure fruc-fruct-glu-gal, and thus comprises 50% fruc, 25% gal and 25% glu. Oligosaccharide 2 (OL2) has the structure fruc-fruc-glu, and thus comprises 66% fruc, 33% glu. The different oligosaccharides thus have a homology of 75% (50% fruc + 25% glu).

In a preferred embodiment, the present composition comprises galactooligosaccharides and at least one selected from the group consisting of fructooligosaccharides and inulin.

Each of the present oligosaccharides preferably comprises at least 66%, more preferably at least 90% monose units selected from the group consisting of mannose, arabinose, fructose, fucose, rhamnose, galactose, β-D-galactopyranose, ribose, glucose, xylose, uronic acid and derivatives thereof, calculated on the total number of monose units contained therein.

According to a further embodiment at least one of the oligosaccharides of the present composition is selected from the group consisting of fructans, fructooligosaccharides, indigestible dextrins galactooligosaccharides (including transgalactooligosaccharides), xylooligosaccharides, arabinooligosaccharides, glucooligosaccharides, mannooligosaccharides, fucooligosaccharides, acidic oligosaccharides (see below, e.g. uronic acid oligosaccharides such as pectin hydrolysate) and mixtures thereof. Preferably the present composition comprises at least one, preferably at least two, of the oligosaccharides selected from the group consisting of fructooligosaccharides or inulin, galactooligosaccharides and pectin hydrolysate.

For good mucus quantity and quality, the present composition preferably comprises at least one oligosaccharide, which comprises at least 66% galatose or fructose as a monose unit. In a preferred embodiment the composition comprises at least one oligosaccharide which comprises at least 66% galatose as a monose unit and at least one oligosaccharide which comprises at least 66% fructose as a monose unit. In a particularly preferred embodiment, the present composition comprises galactooligosaccharide and an oligosaccharide selected from the group consisting of fructooligosaccharides and inulin. Fructooligosaccharides stimulate sulfomucin production in the distal colon of human flora-associated rats (Kleessen et al, (2003) Brit J Nutr 89:597-606) and galactooligosaccharides stimulate the acid mucin production (Meslin et al, Brit. J.Nutr (1993), 69: 903-912)).

For further improvement of mucus layer thickness over the whole area of the colon, at least 10 wt.% of the oligosaccharides in the present composition has a DP of 2 to 5 (i.e. 2, 3, 4 and/or 5) and at least 5 wt.% has a DP of 10 to 60. Preferably at least 50 wt.%, more preferably at least 75 wt.% of the oligosaccharides have a DP of 2 to 9 (i.e. 2, 3, 4, 5, 6, 7, 8, and/or 9), because these are believed to work throughout the ileum and proximal and middle parts of the colon and because the weight percentage of oligosaccharides that needs to be incorporated in the composition to achieve the desired effect is reduced.

Preferably the weight ratios:
a. (oligosaccharides with DP 2 to5) : (oligosaccharides with DP 6,7,8 and/or 9) > 1; and
b. (oligosaccharides with DP 10 to60) : (oligosaccharides with DP 6,7,8 and/or 9) > 1 are both above 1.

Preferably both weight ratios are above 2, even more preferably above 5.

For even further improvement of mucus layer thickness and quality over the whole area of the colon, preferably each of the at least two different oligosaccharides are provided in different chain lengths, preferably at least 10 wt.% of each oligosaccharide based on the total weight of the respective oligosaccharide has a DP of 2 to 5 (i.e. 2, 3, 4 and/or 5) and at least 5 wt.% has a DP between 10 and 60. Preferably at least 50 wt.%, more preferably at least 75 wt.% of the oligosaccharide based on the total weight of that oligosaccharides has a DP between 2 and 10, because these are believed to work throughout in the ileum and proximal and middle parts of the colon.

### Acidic oligosaccharides

To further improve barrier integrity, the present composition preferably includes acidic oligosaccharides with a DP between 2 and 60. The term acid oligosaccharide refers to oligosaccharides comprising at least one acidic group selected from the group consisting of N-acetylneuraminic acid, N-glycoloylneuraminic acid, free or esterified carboxylic acid, sulfuric acid group and phosphoric acid group. The acidic oligosaccharide preferably comprises uronic acid units (i.e. uronic acid polymer), more preferably galacturonic acid units. The acid oligosaccharide may be a homogeneous or heterogeneous carbohydrate. Suitable examples are hydrolysates of pectin and/or alginate. In the intestinal tract, the uronic acid polymers are hydrolysed to uronic acid monomers, which stimulate production of intestinal acetate, which in turn stimulates intestinal mucus secretion (Barcelo et al., Gut 2000; 46:218-224).
Preferably the acid oligosaccharide has the structure I below, wherein the terminal hexose (left) preferably comprises a double bond. The hexose units other than the terminal hexose unit(s) are preferably uronic acid units, even more preferably galacturonic acid units. The carboxylic acid groups on these units may be free or (partly) esterified, and preferably at least 10% is methylated (see below). 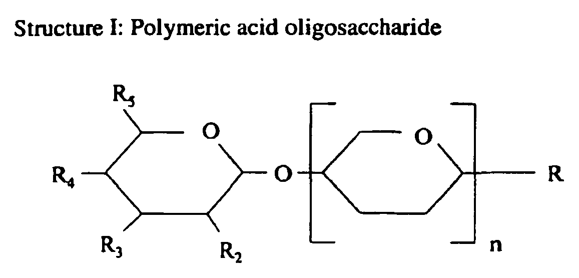 wherein:
R is preferably selected from the group consisting of hydrogen, hydroxy or acid group, preferably hydroxy; and
at least one selected from the group consisting of R₂, R₃, R₄ and R₅ represents N-acetylneuraminic acid, N-glycoloylneuraminic acid, free or esterified carboxylic acid, sulfuric acid group and phosphoric acid group, and the remaining of R₂, R₃, R₄ and R₅ representing hydroxy and/or hydrogen. Preferably one selected from the group consisting of R₂, R₃, R₄ and R₅ represents N-acetylneuraminic acid, N-glycoloylneuraminic acid, free or esterified carboxylic acid, sulfuric acid group or phosphoric acid group, and the remaining represent hydroxy and/or hydrogen. Even more preferably one selected from the group consisting of R₂, R₃, R₄ and R₅ represents free or esterified carboxylic acid and the remaining of R₂, R₃, R₄ and R₅ representing hydroxy and/or hydrogen; and
n is an integer and refers to a number of hexose units (see also Degree of Polymerisation, below), which may be any hexose unit. Suitably n is an integer between 1-5000. Preferably the hexose unit(s) is a uronic acid unit.
Most preferably R₁, R₂ and R₃ represent hydroxy, R₄ represent hydrogen, R₅ represents carboxylic acid, n is any number between 1 and 250, preferably between 1 and 10 and the hexose unit is galacturonic acid.

The detection, measurement and analyses of the preferred acid oligosaccharides as used in the present invention are given in applicants earlier patent application relating to acid oligosaccharides, i.e. WO 0/160378.

For stimulation improvement of mucus layer thickness over the whole area of the colon, the present composition preferably comprises at least 10 wt.% acid oligosaccharides with a DP of 2 to 5 (i.e. 2, 3, 4 and/or 5) and at least 5 wt.% acid oligosaccharides with a DP between 10 and 60, said wt.% being based on the total weight of the oligosaccharides.

The acid oligosaccharides used in the invention are prepared from pectin, pectate, alginate, chondroitine, hyaluronic acids, heparine, heparane, sialoglycans, fucoidan, fucooligosaccharides or carrageenan, preferably from pectin and/or alginate.

### Content of oligosaccharide

When in ready-to-feed liquid form, the present composition preferably comprises 0.1 to 100 grams indigestible oligosaccharide per liter, more preferably between 0.5 and 50 grams per liter even more preferably between 1 and 25 grams per liter. A too high content of oligosaccharides may cause discomfort due to excessive fermentation, while a very low content may result in an insufficient mucus layer.
The weight ratio of the at least two different oligosaccharides is preferably between 1 and 10, more preferably between 1 and 5. These weight ratios stimulate mucin production of different types at different sites in the intestine optimally.

The oligosaccharide is preferably included in the present composition according to the invention in an amount exceeding 0.1 wt.%, preferably exceeding 0.2 wt.%, more preferably exceeding 0.5 wt.% and even more preferably exceeding 1 wt.% based on the total dry weight of the composition. The present composition preferably has an oligosaccharide content below 20-wt.%, more preferably below 10-wt.% even more preferably below 5-wt.%.

Addition of nucleotides and/or nucleosides to the present composition further improves gut mucosal barrier function, particularly as it inhibits and/or or reduces the incidence of bacterial translocation and decreases intestinal injury. Hence, the present composition preferably also comprises between 1 and 500 mg nucleosides and/or nucleotides per 100 gram of the dry formula, even more preferably between 5 and 100 mg.

### Application

The present composition can be advantageously used in for improving barrier integrity in mammals, particularly humans. The present composition can also be advantageously used for the treatment or prevention of diseases associated with reduced barrier integrity. The present composition is preferably administered orally.

For the ill and infants, the present composition is preferably combined with complete nutrition, including protein, carbohydrate and fat. The present composition is advantageously administered to infants with the age between 0 and 2 years. The composition may be administered to patients which suffer from an impaired barrier integrity and healthy patients. The present composition is advantageously used in a method for providing the nutritional requirements of a premature infant (an infant born before 37 weeks gestation).

The present composition can also be advantageously used in a for treatment and/or prevention of intestinal damage by administering the present composition to the patient prior to or after a medical treatment, which may cause intestinal damage. Such medical treatment may for example be surgery or enteral medicine treatment (e.g. antibiotic, analgesic, NSAID, chemotherapeutic agents etc).

The present composition can also be advantageously used to treat or prevent diseases wherein intestinal barrier disruption is underlying the development of the course of the disease, e.g. in the treatment or prevention of chronic inflammatory diseases, particularly inflammatory bowel disease (IBD), irritable bowel syndrome (IBS), celiac disease, pancreatitis, hepatitis, arthritis or diabetes. Furthermore, the invention can be used for providing nutrition to patients that have undergone or are undergoing abdominal surgery and patients that experience postoperative dysfunction of the gut and/or malnourished patients.

In a embodiment of the invention the present composition is advantageously administered to patients suffering from acquired immune deficiency syndrome (AIDS) and/or patients which are infected with the human immunodeficiency virus (HIV), e.g. in the treatment of AIDS and/or HIV infection. Said treatment comprises the oral administration of the present composition, preferably combined with nutrients selected from the group consisting of carbohydrate, protein and fat. The compositions according to the invention are particularly useful for patients with a CD4⁺ T cell count that is below the critical level of around 700 cells/µl blood, when generally Highly Active Antiretroviral Therapy (HAART) therapy is not yet needed, but when patients do already run the risk of developing or even experience one or more problems that can be related to intestinal barrier integrity, in particular dysfunction of intestinal barrier integrity. Hence, in a further aspect the present invention helps stopping or slowing down the reduction in CD4+ T cell count or improving the CD4+ T cell count in patients suffering from HIV and/or AIDS. In one embodiment the invention comprises administering the present composition to a patient, said patient being a human subject having a CD4+ T-lymphocyte cell count between 200 to 700 cells/µl blood. In yet another embodiment the invention comprises administering the present composition to a patient, said patient not being treated by Highly Active Antiretroviral Therapy. In a particular embodiment the patient has a CD4+ T-lymphocyte cell count between 200 to 700 cells/µl blood and is not being treated by Highly Active Antiretroviral Therapy.

Furthermore, the invention can also be used to treat or prevent complications resulting from reduced barrier integrity, particularly in the treatment and/or prevention of diarrhea, particularly infant diarrhea. Due to the reduced incidence in infant diarrhea, the present composition can also be advantageously used to reduce diaper rash.

Administering the present composition reduces passage of dietary and microbial antigens, particularly food allergens, from the intestinal lumen into the mucosal or systemic circulation, and hence can be advantageously used in the treatment or prevention of allergy and/or allergic reaction, particularly in the treatment or prevention of food allergy, e.g. allergic reaction resulting from the ingestion of foodstuff.

It was also found by the present inventors that EPA, DHA and/or ARA are capable of reducing the effects of IL-4 on intestinal permeability. Hence, the present composition can be used for the treatment and/or prevention of diseases wherein intestinal IL-4 concentration is increased (e.g. allergic diseases), such as atopic dermatitis. The present composition is preferably provided as a packaged powder or packaged ready-to-feed formula. To prevent spoilage of the product, packaging size of ready-to-feed formula preferably does not exceed one serving, e.g. preferably does not exceed 500 ml; and packaging size of the present composition in powder form preferably does not exceed 250 servings. Suitable packaging sizes for the powder are 2000 grams or less, preferably per 1000 grams or less.

The packaged products provided with labels that explicitly or implicitly direct the consumer towards the use of said product in accordance with one or more of the above or below purposes are encompassed by the present invention. Such labels may for example make reference to the present method for preventing allergic reaction to food allergens by including wording like "reduced food sensitivity", "improving intestinal tolerability", "improved food tolerance" or similar wording. Similarly, reference to the present method for treating and/or preventing allergy may be made by incorporating terminology equivalent to "improved resistance" or "reduced sensitivity".

### Formula's

It was found that the present composition could be advantageously applied in food, such as baby food and clinical food. Such food preferably comprises lipid, protein and carbohydrate and is preferably administered in liquid form. The term "liquid food" as used in the present invention includes dry food (e.g. powders), which are accompanied with instructions as to admix said dry food mixture with a suitable liquid (e.g. water).

Hence, the present invention also relates to a nutritional composition which preferably comprises between 5 and 50 en% lipid, between 5 and 50 en% protein, between 15 and 90 en% carbohydrate and the present combination of oligosaccharides and LC-PUFA's. Preferably the present nutritional composition preferably contains between 10 and 30 en% lipid, between 7.5 and 40 en% protein and between 25 and 75 en% carbohydrate (en% is short for energy percentage and represents the relative amount each constituent contributes to the total caloric value of the preparation).

Preferably a combination of vegetable lipids and at least one oil selected from the group consisting of fish oil and omega-3 vegetable, algae or bacterial oil is used.

The proteins used in the nutritional preparation are preferably selected from the group of non-human animal proteins (such as milk proteins, meat proteins and egg proteins), vegetable proteins (such as soy protein, wheat protein, rice protein, and pea protein), free amino acids and mixtures thereof. Cow milk derived nitrogen source, particularly cow milk protein proteins such as casein and whey proteins are particularly preferred.

Stool irregularities (e.g. hard stools, insufficient stool volume, diarrhoea) is a major problem in many babies and ill subjects that receive liquid foods. It was found that stool problems may be reduced by administering the present oligosaccharides in liquid food which have an osmolality between 50 and 500 mOsm/kg, more preferably between 100 and 400 mOsm/kg.

In view of the above, it is also important that the liquid food does not have an excessive caloric density, however still provides sufficient calories to feed the subject. Hence, the liquid food preferably has a caloric density between 0.1 and 2.5 kcal/ml, even more preferably a caloric density of between 0.5 and 1.5 kcal/ml, most preferably between 0.6 and 0.8 kcal/ml.

### EXAMPLES

### Example 1: Effect of LC-PUFA on barrier integrity

Monolayers (MC) of intestinal epithelial cell lines T84 (American Type Culture Collection (ATTC), Manassas, USA) were cultured on transwell filters (Corning, Costar BV, The Netherlands) allowing both mucosal and serosal sampling and stimulation of human intestinal epithelial cells. Two weeks post confluency the monolayers were incubated in the luminal compartment with polyunsaturated fatty acids ARA (arachidonic acid; 5,8,11,14-eicosatetraenoic acid), DHA (cis-4,7,10,13,16,19 docosahexaenoic acid), EPA (eicosapentaenoic acid) or control palmitic (C 16:0) acid (Palm) (Sigma, St. Louis, USA). The latter procedure was chosen to mimic the in vivo administration route of the dietary compounds. Cells were incubated with ARA, DHA, EPA, GLA or palmitic acid for 0, 24, 48 and 72 hr at different concentrations (10 µM and 100 µM). Experiments were performed to evaluate basal barrier integrity. The epithelial barrier function was determined by measuring the transepithelial resistance (TER, Ω.cm²) was measured by epithelial volt-ohm meter (EVOM; World Precision Instruments, Germany) and permeability for 4kD FITC dextran (paracellular permeability marker, Sigma, USA). Resistance (. Epithelial permeability for 4 kDa FITC-dextran was determined as follows. Prior to dextran fluxes the medium was refreshed with culture medium without phenol red for one hour followed by addition of 5 µl (stock 100 mg/ml) 4 kDa FITC-dextran to the lumenal compartment. After 30 min incubation 100 µl sample was collected from the serosal compartment and the fluorescent signal measured at excitation wavelength 485 nm and emission 520 nm (FLUOstar Galaxy®, BMG Labtechnologies, USA). FITC-dextran fluxes were calculated as pmol FITC-dextran/cm²/h. Statistical analyses were performed using the ANOVA (SPSS version 10).
Results of the effect of fatty acids (100 µM) on spontaneous barrier integrity after 72 hr incubation are given in Table 1. Table 1 shows that the LC-PUFA's ARA, EPA, GLA and DHA reduce the molecular flux and improve epithelial resistance. In contrast the control experiments show that palmitic acid has the opposite effects, i.e. compromises barrier integrity. These results are indicative for the advantageous use of EPA, DHA , GLA and ARA, and in particularly ARA in the composition according to the present invention and for use in a method according to the present invention, e.g. in a method for improving barrier integrity. These result further support the synergistic effects of the present combination of fatty acids and indigestible oligosaccharides.

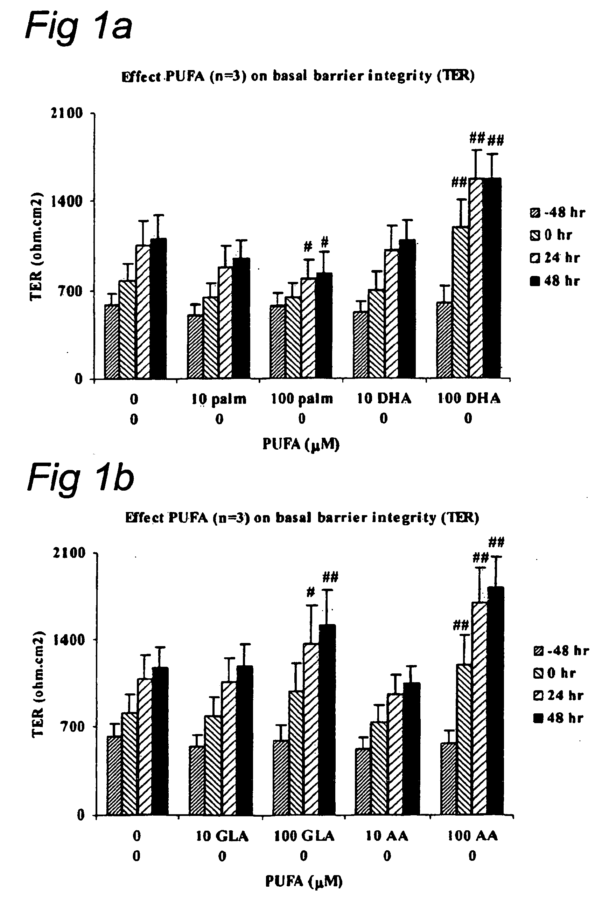

Figure 1 shows the time and dose (10µM and 100µM) dependent effects of various fatty acids (palmitic acid, DHA, GLA, and AA) on basal barrier integrity (TER). Figure 1 shows that the LC-PUFA's AA, DHA, and GLA, improve the epithelial barrier integrity as reflected by increased resistance (TER). These results are indicative for the advantageous use of EPA, DHA, GLA and ARA, in particularly ARA, in the composition according to the present invention and for for improving barrier integrity. These results further support the synergistic effects of the present combination of fatty acids and indigestible oligosaccharides.

**Table 1**

| Ingredient (LC-PUFA) | Flux | Resistance (TER) |
|---|---|---|
| Control | 79 | 1090 |
| Palmitic acid | 161 | 831 |
| DHA | 72 | 1574 |
| ARA | 28 | 1816 |
| EPA | 65 | 1493 |

### Example 2: Effect of LC-PUFA on IL-4 mediated barrier disruption

Monolayers (MC) of intestinal epithelial cell lines T84 (ATCC, USA) were cultured on transwell filters (Coming, Costar BV, The Netherlands) allowing both mucosal and serosal sampling and stimulation of human intestinal epithelial cells. Two weeks post confluency the monolayers were incubated in the presence of IL-4 (2 ng/ml, serosal compartment, Sigma, USA ) with or without polyunsaturated fatty acids ARA, DHA, GLA, EPA, or control palmitic acid (10 µM or 100 µM, mucosal compartment, Sigma, St. Louis, USA). Cells were pre-incubated with GLA, ARA, DHA, EPA, or palmitic acid for 48 hr prior to the IL-4 incubation. The co-incubation of PUFA's and palmetic acid with IL-4 was continued for another 48 hr; while culture medium and additives were changed every 24 hr. The epithelial barrier function was determined by measuring the transepithelial resistance (TER) and permeability as described in example 1. Statistical evaluation was performed as described in example 1.
Results of the effect of GLA, ARA, DHA, EPA and palmitic acid (100 µM) on IL-4 mediated barrier disruption are given in Table 2. Table 2 shows that the LC-PUFA's GLA, ARA, DHA and EPA inhibit the increased flux caused by IL-4. In contrast palmetic acid had a detrimental effect and decreased barrier disruption compared to control. These results are indicative for the advantageous use of GLA, ARA, DHA, and EPA in clinical and infant nutrition formulations to prevent or reduce IL-4 mediated barrier disruption, e.g. as occurs in food or cows milk allergy. These result further support the synergistic effects of the present combination of fatty acids and indigestible oligosaccharides.

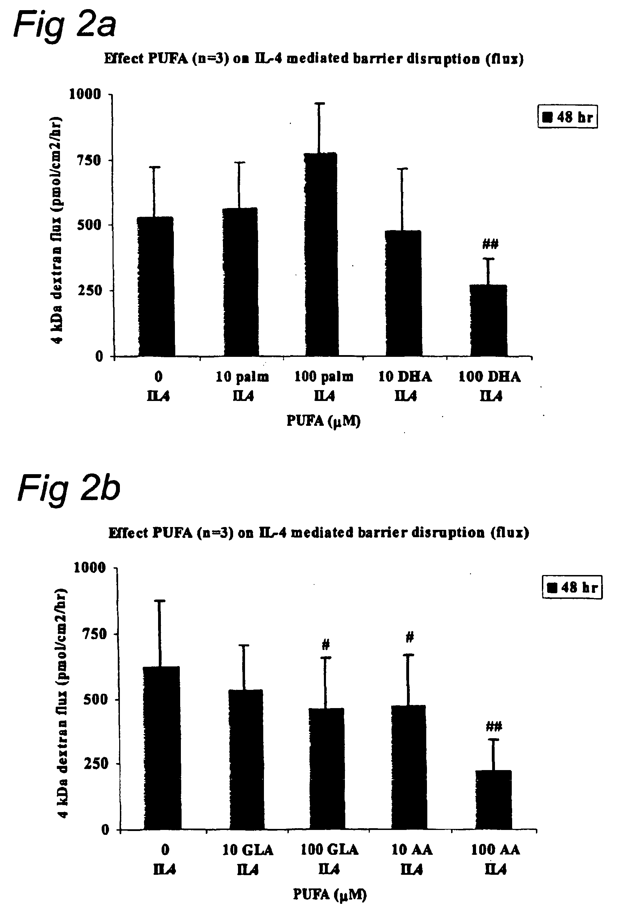

Figure 2 gives the time and dose (10µM and 100µM) dependent protective effects of various FA's (palmitic acid, DHA, GLA, and ARA) on IL-4 mediated barrier destruction (Flux). Figure 2 shows that ARA, DHA and GLA protect against IL-4 mediated barrier disruption as reflected by decreased 4kD dextran flux. These results are indicative for the advantageous use of GLA, ARA, DHA and EPA in clinical and infant nutrition formulations to prevent or reduce IL-4 mediated barrier disruption, e.g. as occurs in food or cows milk allergy. These results further support the synergistic effects of the present combination of fatty acids and indigestible oligosaccharides.

**Table 2**

| Ingredient (PUFA) | Permeability (IL-4 Flux) | Resistance (IL-4 TER) |
|---|---|---|
| Control | 573 | 281 |
| GLA | 360 ↓ | 331 ↑ |
| ARA | 273 ↓ | 337 ↑ |
| EPA | 236 ↓ | 375 ↑ |
| DHA | 304 ↓ | 328 ↑ |

| | | |
|---|---|---|
| ↓ = *reduced permeability by PUFA;* ↑ = *improved resistance by PUFA* | | |

### Example 3: Effect of oligosaccharides on acetate production

Micro-organisms were obtained from fresh faeces from bottle fed babies. Fresh faecal material from babies ranging 1 to 4 month of age was pooled and put into preservative medium within 2 h. As substrate either prebiotics (TOS; TOS/inulin (HP) mixture in a 9/1 (w/w) ratio; inulin; oligofructose(OS)/inulin mixture in a 1/1 (w/w) ratio, or none (blanc) were used. The transgalactooligosaccharides (TOS) were obtained from Vivinal GOS, Borculo Domo Ingredients, Zwolle, The Netherlands and comprises as indigestible oligosaccharides: 33 wt.% disaccharides, 39 wt.% trisaccharides, 18 wt.% tetrasaccharides, 7 wt.% pentasaccharides and 3 wt.% hexa-, hepta- en octasaccharides. The inulin (HP) Orafti active food ingredients, Tienen, Belgium, i.e. Raftiline HP®, with an average DP of 23.**Media**: McBain & MacFarlane medium: buffered peptone water 3.0g/l, yeast extract 2.5 g/l. mucin (brush borders) 0.8 g/l, tryptone 3.0g/l, L-Cysteine-HCl 0.4 g/l, bile salts 0.05 g/l, K2HPO4.3H2O 2.6 g/l, NaHCO3 0.2 g/l, NaCl 4.5 g/l, MgSO4.7H2O 0.5 g/l, CaCl2 0.228 g/l, FeSO4.7H2O 0.005 g/l. Fill 500 ml Scott bottles with the medium and sterilized 15 minutes at 121 °C. **Buffered medium:** K2HPO4.3H2) 2.6 g/l, NaHCO3 0.2 g/l, NaCl 4.5 g/l, MgSO4.7H2O, 0.5 g/l, CaCl2 0.228 g/l, FeSO4.7H2O 0.005 g/l. Adjust to pH 6.3 ± 0.1 with K2HPO4 or NaHC03. Fill 500ml Scott bottles with the medium and sterilized 15 minutes at 121 °C.
**Preservative medium:** Buffered peptone 20.0 g/l, L-Cysteine-HCl 0.5 g/l, Sodium thioglycollate 0.5 g/l, resazurine tablet 1 per litre, adjust to pH 6.7 ± 0.1 with 1 M NaOH or HCl. Boiled in microwave. Serum bottles were filled with 25 ml medium and sterilized for 15 minutes at 121 °C.
Fresh faecal samples were mixed with preservative medium and stored for several hours at 4 °C. The preserved solution of faeces was centrifuged at 13,000 rpm for 15 minutes, supernatant removed and faeces mixed with McBain & Mac Farlane medium in a weight ratio of 1:5. Of this faecal suspension 3 ml were combined with 85 mg glucose or prebiotic or with no addition (blanc) in a bottle and mixed thoroughly. A t=0 sample was withdrawn (0.5 ml). 2.5 ml of the resulting suspension is brought in a dialysis tube in a 60 ml bottle filled with 60 ml of the buffered medium. The bottle was closed well and incubated at 37°C. Samples were taken from the dialysis tube (0.2 ml) or dialysis buffer (1.0 ml) with a hypodermic syringe after 3, 24, and 48 hours and immediately put it on ice to stop fermentation. The experiment was carried out using the following samples:
1) 85mg TOS
2) 85 mg inulin
3) 85mg TOS/inulin in a ratio of 9/1 (w/w) and
4) 85mg OS/inulin in a ratio of 1/1 (w/w).

SCFA (acetate, propionate, butyrate) were quantitated using a Varian 3800 gas chromatograph (GC) (Varian Inc., Walnut Creek, U.S.A.) equipped with a flame ionisation detector. 0.5 µl of the sample was injected at 80 °C in the column (Stabilwax, 15 x 0.53 mm, film thickness 1.00 µm, Restek Co., U.S.A.) using helium as a carrier gas (3.0 psi). After injection of the sample, the oven was heated to 160 °C at a speed of 16 °C/min, followed by heating to 220 °C at a speed of 20 °C/min and finally maintained at 220 °C for 1.5 minutes. The temperature of the injector and detector was 200 °C. 2-ethylbytyric acid was used as an internal standard.

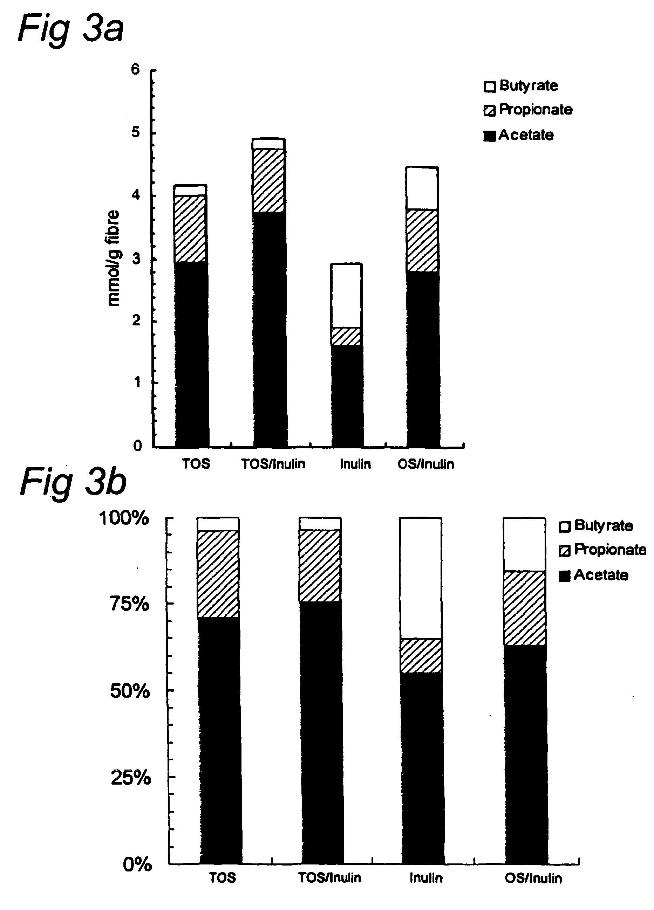

Figure 3 depicts the absolute (Figure 3A) and relative SCFA profile (Figure 3B) resulting from fermenting the different oligosaccharides. Figure 3A shows that a mixture of two different oligosaccharides (TOS/Inulin), wherein the two distinct oligosaccharides have a homology in monose units below 90 and a different chain length results in a significantly and synergistically increased amount of SCFA (particularly acetate) per gram fiber than single components. Figure 3B shows that the addition of a combination of TOS/Inulin favored a higher proportion of the beneficial acetate (B). The acetate production in vivo translates to improved mucus production by goblet cells and a measure for intestinal mucus layer thickness (see example 4). These results are indicative for the advantageous use of the present composition.

### Example 4: Effects of SCFA on mucus production.

Monolayers of intestinal epithelial T84 cells (ATCC, USA) cells were cultured in 24 or 96 wells tissue culture plates (Corning B.V.). T84 were incubated with the short chain fatty acids acetate, proprionate and butyrate (SCFA, Merck, USA) for 24 h in a concentration range of 0.025-4.0 mM. Supernatants and/or cells were collected and MUC-2 (mucin) expression determined. A dotblot technique was used to determine MUC-2 expression in the cell cultures, since mucins are extremely large glycoproteins (over 500 kDa) which makes them difficult to handle in western blotting techniques. The method was validated using pre-immune serum (T84 stained negative), CCD-18Co (ATCC, USA) negative control cells and bovine serum albumin (BSA). Cell samples were collected in Laemmli (protein isolation buffer) and protein determination performed using a microprotein assay (Biorad, USA) according to the manufacturers protocol. Samples (0.3-0.7-1.0 µg/2 µl) were dotted on nitrocellulose membranes (Schleicher & Schuell, Germany). Membranes were blocked in TBST/5% Protivar (Nutricia, The Netherlands) followed by 1 h incubation with anti-MUC-2 antibody (kindly donated by Dr. Einerhand, Erasmus University, Rotterdam, The Netherlands). After washing, blots were incubated with goat anti-rabbit-HRP (Santacruz Biotechnology, USA) and for substrate detection ECL (Roche Diagnostics, The Netherlands) was used. Densitometry was performed using the Lumi-Imager (Boehringer Mannheim B.V., The Netherlands) and the signal was expressed in light units (BLU). BLU's were also expressed relative to control incubations (%BLU). To compare the stimulatory effect of SCFA on MUC-2 expression basal MUC-2 expression levels were deducted.

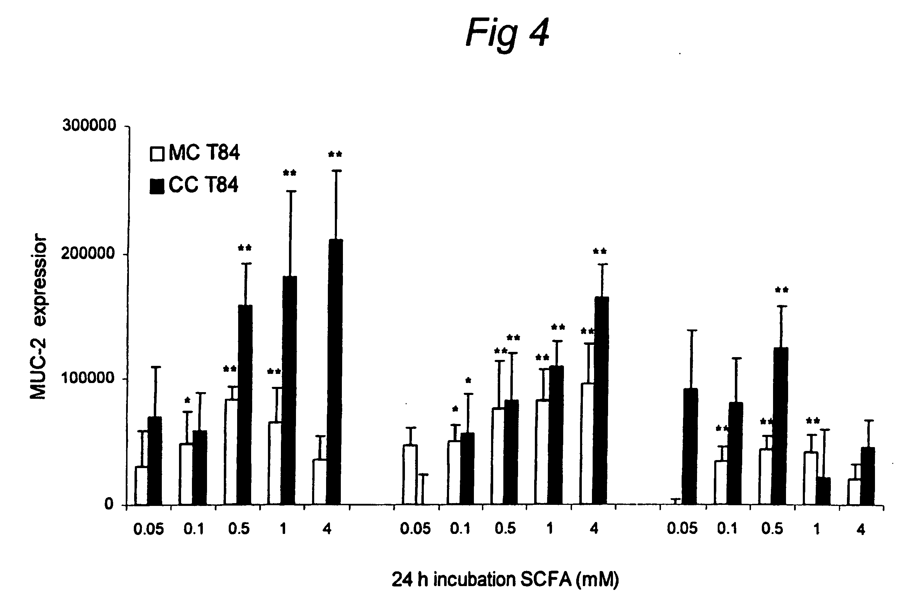

Figure 4 shows the differential effects of SCFA (acetate, proprionate, butyrate) on MUC-2 expression in intestinal epithelial cells (MC T84) and epithelial-mesenchymal cell co-cultures (CC T84). Figure 4 also shows that acetate is more potent in stimulating MUC-2 expression (mucus production) as compared to propionate and butyrate. Hence, the present combination of oligosaccharides (which was shown to increase acetate production (see example 3)) is particularly useful for stimulating mucus production and can be advantageously used in a method for stimulating barrier integrity.

### Example 5: Infant milk formula I

Ingredients (per liter), energy 672 Kcal; Protein 15 g; Whey: Casein ratio 60:40; Fat 36 g; Carbohydrate 72 g; Vitamin A 750 RE; Mixed natural carotids 400 IU; Vitamin D 10.6 mcg; Vitamin F 7.4 mg; Vitamin K 67.0 mcg; Vitamin B.sub.1 (thiamin) 1000 mcg; Vitamin B.sub.2 (riboflavin) 1500 mcg; Vitamin B.sub.6 (pyridoxine) 600 mcg; Vitamin B.sub.12 (cyanacobalmine) 2.0 mcg; Niacin 9.0 mcg; Folic Acid 80 mcg; Pantothenic Acid 3000 mcg; Biotin 90 mcg; Vitamin C (ascorbic acid) 90 mg; Choline 100 mg; Inositol 33 mg; Calcium 460 Mg; Phosphorous 333 Mg; Magnesium 64 Mg; Iron 8.0 Mg ; Zinc 6.0 Mg; Manganese 50 mcg; Copper 560 mcg; Iodine 100 mcg; Sodium 160 mg; Potassium 650 mg; Chloride 433 mg and Selenium 14 mcg; wherein the fat content provides includes 3 gram fish oil and 3 grams 40% arachidonic acid oil (DSM Food Specialties, Delft, Netherlands); further comprising 4 gram transgalactooligosaccharides Elix'or^{™} (Borculo Domo Ingredients, Netherlands) and 4 gram Raftiline^{™} (Orafti Active Food Ingredients, Belgium).

### Example 6. Composition of a nutritional bar for the amelioration of HIV/AIDS related symptoms

| **Raw Material** | **g/ day** | **protein** | **carbs** | **fat** | **g/ 100g** |
|---|---|---|---|---|---|
| Milk protein | 20.00 | 15.00 | 2.10 | 0.80 | 21.04 |
| Egg protein | 21.09 | 16.87 | 0.00 | 0.00 | 22.19 |
| borage oil | 4.00 | 0.00 | 0.00 | 4.00 | 4.21 |
| EPA-DHA oil | 6.00 | 0.00 | 0.00 | 6.00 | 6.31 |
| Galacto-oligosaccharides | 15.38 | 0.00 | 4.78 | 0.00 | 16.18 |
| Inuline | 0.79 | 0.00 | 0.00 | 0.00 | 0.83 |
| Pectin hydrol | 8.54 | 0.11 | 0.09 | 0.00 | 8.98 |
| Fructosestroop | 15.40 | 0.00 | 11.92 | 0.00 | 16.20 |
| glycerine | 3.85 | 0.00 | 3.83 | 0.00 | 4.05 |
| | | | | | |
| *SUM* | *95.05* | *31.98* | *22.72* | *10.80* | *100.00* |

| | **per day kcal** | **En%** | **per 100g kcal** |
|---|---|---|---|
| energy protein | 128 | 40.5 | 135 |
| energy carbs | 91 | 28.8 | 96 |
| energy fat | 97 | 30.8 | 102 |

## Claims

1. Use of polyunsaturated fatty acids for the manufacture of a composition for the treatment of a patient infected with human immunodeficiency virus (HIV), said composition comprising:
a. eicosapentaenoic acid (EPA), docosahexaenoic acid (DHA) and arachidonic acid (ARA), wherein the content of long chain polyunsaturated fatty acid with 20 and 22 carbon atoms is at least 15 wt.% and does not exceed 85 wt.% of the total fat content and wherein the ARA content is at least 0.1 wt. % of the total fat; and
b. at least two distinct oligosaccharides (OL1 and OL2), wherein the two distinct oligosaccharides have a homology in monose units below 90 %.

2. Use according to claim 1 wherein the composition further comprises gamma-linolenic acid (GLA).

3. Nutritional composition comprising:
a. EPA, DHA and ARA, wherein the content of long chain polyunsaturated fatty acid with 20 and 22 carbon atoms is at least 15 wt.% and does not exceed 85 wt.% of the total fat content and wherein the ARA content is at least 0.1 wt. % of the total fat;
b. at least two distinct oligosaccharides (OL1 and OL2), wherein the two distinct oligosaccharides have a homology in monose units below 90 %; and
c. an acidic oligosaccharide with a DP between 2 and 60, prepared from pectin, pectate, alginate, chondroitine, hyaluronic acids, heparine, heparane, sialoglycans, fucoidan, fucooligosaccharides or carrageenan.

4. Composition according to claim 3, comprising galactooligosaccharide and a fructan selected from the group consisting of fructooligosaccharides, inulin and mixtures thereof.

5. Composition according to claim 3 or 4, wherein at least 10 wt.% of the oligosaccharide has a degree of polymerisation (DP) of 2 to 5 and at least 5 wt.% has a DP of between 10 and 60.

6. Composition according to any one of claims 3-5, comprising 7.5 to 12.5 energy % protein; 40 to 55 energy % carbohydrates; and 35 to 50 energy % fat, wherein said protein comprises a member selected from the group consisting of hydrolyzed milk protein, vegetable protein and/or amino acids.

7. Composition according to any one of claims 3-6, said composition having a caloric content of 0.6 to 0.8 kcal/ml; an osmolality of 50 to 500 mOsm/kg; and a viscosity below 50 mPas.

8. Composition according to any one of claims 3-7, wherein the composition further comprises GLA.

9. Composition according to any one of claims 3-8, for use as a medicament.

10. Use according to claim 1, wherein the patient is a human subject having a CD4+ T-lymphocyte cell count between 200 to 700 cells/µl blood and wherein said patient is not being treated by Highly Active Antiretroviral Therapy.

11. Use of a composition according to any one of claims 3-9 for the manufacture of a medicament for the treatment or prevention of diarrhea in a mammal.

## Patentansprüche

1. Verwendung von mehrfach ungesättigten Fettsäuren für die Herstellung einer Zusammensetzung für die Behandlung eines Patienten, der mit humanem Immundefizienzvirus (HIV) infiziert ist, wobei die Zusammensetzung umfasst:
a. Eicosapentaensäure (EPA), Docosahexaensäure (DHA) und Arachidonsäure (ARA), wobei der Gehalt an langkettiger mehrfach ungesättigter Fettsäure mit 20 und 22 Kohlenstoffatomen wenigstens 15 Gew.-% beträgt und 85 Gew.-% nicht überschreitet, bezogen auf den Gesamtfettgehalt, und wobei der Gehalt an ARA mindestens 0,1 Gew.-% beträgt, bezogen auf den Gesamtfettgehalt; und
b. wenigstens zwei voneinander verschiedene Oligosaccharide (OL1 und OL2), wobei die zwei voneinander verschiedenen Oligosaccharide eine Homologie von Monoseeinheiten unter 90 % aufweisen.

2. Verwendung nach Anspruch 1, wobei die Zusammensetzung außerdem Gamma-Linolensäure (GLA) umfasst.

3. Nahrungszusammensetzung, umfassend:
a. EPA, DHA und ARA, wobei der Gehalt an langkettiger mehrfach ungesättigter Fettsäure mit 20 und 22 Kohlenstoffatomen wenigstens 15 Gew.-% beträgt und 85 Gew.-% nicht überschreitet, bezogen auf den Gesamtfettgehalt, und wobei der Gehalt an ARA mindestens 0,1 Gew.-% beträgt, bezogen auf den Gesamtfettgehalt; und
b. wenigstens zwei voneinander verschiedene Oligosaccharide (OL1 und OL2), wobei die zwei voneinander verschiedenen Oligosaccharide eine Homologie von Monoseeinheiten unter 90 % aufweisen;
c. ein saures Oligosaccharid mit einem DP zwischen 2 und 60, hergestellt aus Pektin, Pektat, Alginat, Chondroitin, Hyaluronsäuren, Heparin, Heparan, Sialoglycanen, Fucoidan, Fucooligosacchariden oder Carrageen.

4. Zusammensetzung nach Anspruch 3, umfassend Galactooligosaccharid und ein Fructan, ausgewählt aus der Gruppe bestehend aus Fructooligosacchariden, Inulin und Mischungen davon.

5. Zusammensetzung nach Anspruch 3 oder 4, wobei wenigstens 10 Gew.-% des Oligosaccharids einen Polymerisationsgrad (DP) von 2 bis 5 aufweisen und wenigstens 5 Gew.-% einen DP zwischen 10 und 60 aufweisen.

6. Zusammensetzung nach einem der Ansprüche 3-5, umfassend 7,5 bis 12,5 Energieprozent Protein; 40 bis 55 Energieprozent Kohlenhydrate; und 35 bis 50 Energieprozent Fett, wobei das Protein ein Mitglied, ausgewählt aus der Gruppe bestehend aus hydrolysiertem Milchprotein, pflanzlichem Protein und/oder Aminosäuren, umfasst.

7. Zusammensetzung nach einem der Ansprüche 3-6, wobei die Zusammensetzung einen Kaloriengehalt von 0,6 bis 0,8 kcal/ml; eine Osmolalität von 50 bis 500 mOsm/kg; und eine Viskosität unter 50 mPas aufweist.

8. Zusammensetzung nach einem der Ansprüche 3-7, wobei die Zusammensetzung außerdem GLA umfasst.

9. Zusammensetzung nach einem der Ansprüche 3-8 zur Verwendung als ein Medikament.

10. Verwendung nach Anspruch 1, wobei der Patient ein menschliches Subjekt mit einer CD4+ T-Lymphozytenzellzahl zwischen 200 und 700 Zellen/µl Blut ist und wobei der Patient nicht durch eine hochaktive antiretrovirale Therapie behandelt wird.

11. Verwendung einer Zusammensetzung nach einem der Ansprüche 3-9 für die Herstellung eines Medikaments für die Behandlung oder Verhütung von Diarrhö in einem Säuger.

## Revendications

1. Utilisation d'acides gras polyinsaturés pour la fabrication d'une composition destinée au traitement d'un patient infecté par le virus de l'immunodéficience humaine (VIH), ladite composition comprenant :
a. de l'acide éicosapentaénoïque (EPA), de l'acide docosahexaénoïque (DHA) et de l'acide arachidonique (ARA), la teneur en acide gras polyinsaturé à chaîne longue avec 20 et 22 atomes de carbone étant d'au moins 15 % en poids et ne dépassant pas 85 % en poids de la teneur totale en lipides, et la teneur en ARA étant au moins 0.1 % en poids de la teneur totale en lipides;
b. au moins deux oligosaccharides (OL1 et OL2) distincts, les deux oligosaccharides distincts présentant une homologie en unités de monose au-dessous de 90 %.

2. Utilisation selon la revendication 1, où la composition comprend en outre de l'acide gamma-linolénique (GLA).

3. Composition nutritive comprenant :
a. de l'EPA, du DHA et de l'ARA, la teneur en acide gras polyinsaturé à chaîne longue avec 20 et 22 atomes de carbone étant d'au moins 15 % en poids et ne dépassant pas 85 % en poids de la teneur totale en lipides, et la teneur en ARA étant au moins 0.1 % en poids de la teneur totale en lipides; et
b. au moins deux oligosaccharides (OL1 et OL2) distincts, les deux oligosaccharides distincts présentant une homologie en unités de monose au-dessous de 90 % ;
c. un oligosaccharide acide présentant un DP entre 2 et 60, préparé à partir de pectine, pectate, alginate, chondroïtine, acides hyaluroniques, héparine, héparane, sialoglycanes, fucoïdane, fuco-oligosaccharides ou carraghénane.

4. Composition selon la revendication 3, comprenant un galacto-oligosaccharide et un fructane choisi dans le groupe constitué de fructo-oligosaccharides, d'inuline et de mélanges de ceux-ci.

5. Composition selon la revendication 3 ou 4, dans laquelle au moins 10 % en poids de l'oligosaccharide présente un degré de polymérisation (DP) de 2 à 5 et au moins 5 % en poids présente un DP entre 10 et 60.

6. Composition selon l'une quelconque des revendications 3 à 5, comprenant 7,5 à 12,5 % en énergie de protéines, 40 à 55 % en énergie de glucides, et 35 à 50 % en énergie de lipides, où lesdites protéines comprennent un membre choisi dans le groupe constitué de protéines hydrolysées du lait, et de protéines et/ou d'acides aminés de végétaux.

7. Composition selon l'une quelconque des revendications 3 à 6, ladite composition ayant une teneur calorique de 0,6 à 0,8 kcal/ml, une osmolalité de 50 à 500 mOsm/kg et une viscosité inférieure à 50 mPa.

8. Composition selon l'une quelconque des revendications 3 à 7, où la composition comprend en outre du GLA.

9. Composition selon l'une quelconque des revendications 3 à 8, pour une utilisation en tant que médicament.

10. Utilisation selon la revendication 1, où le patient est un sujet humain ayant un nombre de lymphocytes T CD4+ entre 200 et 700 cellules/· l de sang et où ledit patient n'est pas traité par thérapie antirétrovirale à haute efficacité.

11. Utilisation d'une composition selon l'une quelconque des revendications 3 à 9, pour la fabrication d'un médicament destiné au traitement ou à la prévention de la diarrhée chez un mammifère.
